**Europäisches Patentamt**

**European Patent Office**

**Office européen des brevets**

(19)

(11) Publication number: **0 097 476**
**B1**

(12) **EUROPEAN PATENT SPECIFICATION**

(45) Date of publication of patent specification: **13.09.89**

(51) Int. Cl.⁴: **A 61 K 7/16**

(21) Application number: **83303417.6**

(22) Date of filing: **14.06.83**

(60) Divisional applications 88103994, 88103995, 88103996 filed 14.03.88.

(54) **Oral compositions.**

(30) Priority: **22.06.82 US 391040**

(43) Date of publication of application:
**04.01.84 Bulletin 84/01**

(45) Publication of the grant of the patent:
**13.09.89 Bulletin 89/37**

(84) Designated Contracting States:
**AT BE CH DE FR GB IT LI NL SE**

(56) References cited:
**US-A-2 876 167**
**US-A-3 070 510**
**US-A-3 112 247**
**US-A-3 137 632**
**US-A-3 608 068**
**US-A-3 927 201**
**US-A-3 927 202**
**US-A-4 296 096**
**US-A-4 323 551**

(73) Proprietor: **THE PROCTER & GAMBLE COMPANY**
**301 East Sixth Street**
**Cincinnati Ohio 45201 (US)**

(72) Inventor: **Parran, John Joseph Jr.**
**1155 Meredith Drive**
**Cincinnati, Ohio 45231, (US)**
Inventor: **Sakkab, Nabil Yaqub,**
**1403 Oakridge Road,**
**Loveland Ohio 45040, (US)**

(74) Representative: **Brooks, Maxim Courtney et al**
**Procter & Gamble (NTC) Limited Whitley Road**
**Longbenton**
**Newcastle-upon-Tyne NE12 9TS (GB)**

Courier Press, Leamington Spa, England.

## Description

The present invention relates to oral compositions in the form of toothpastes which provide an anticalculus benefit.

Dental calculus, or tartar as it is sometimes called, is a deposit which forms on the surfaces of the teeth at the gingival margin. Supragingival calculus appears principally in the areas near the orifices of the salivary ducts; e.g., on the lingual surfaces of the lower anterior teeth and on the buccal surfaces of the upper first and second molars, and on the distal surfaces of the posterior molars.

Mature calculus consists of an inorganic portion which is largely calcium phosphate arranged in a hydroxyapatite crystal lattice structure similar to bone, enamel and dentine. An organic portion is also present and consists of desquamated epithelial cells, leukocytes, salivary sediment, food debris and various types of microorganisms.

As the mature calculus develops, it becomes visibly white or yellowish in color unless stained or discolored by some extraneous agent. In addition to being unsightly and undesirable from an aesthetic standpoint, the mature calculus deposits are constant sources of irritation of the gingiva.

A wide variety of chemical and biological agents have been suggested in the art to retard calculus formation or to remove calculus after it is formed. Mechanical removal of this material periodically by the dentist is, of course, routine dental office procedure.

The chemical approach to calculus inhibition generally involves chelation of calcium ion and/or crystal growth inhibition which prevents the calculus from forming and/or breaks down mature calculus by removing calcium.

The prior art discloses a number of chelating agents for this purpose. GB—A—490,384 discloses oral compositions containing ethylenediaminetetraacetic acid, nitrilotriacetic acid and related compounds as anticalculus agents. US—A—3,678,154 discloses oral compositions containing certain polyphosphonates and fluoride. US—A—3,737,533 discloses oral compositions containing certain carbonyl diphosphonates.

In addition to the above references, the prior art discloses dentifrices and mouthwashes containing soluble pyrophosphate salts which have been indicated for a variety of purposes. Included among such references are US—A—2,941,926 which discloses dental powders containing chlorophyll and pyrophosphate salts.

US—A—3,137,632 discloses toothpastes containing pyrophosphate salts. US—A—3,927,201 and US—A—3,927,202 disclose toothpastes which utilize soluble pyrophosphates as abrasives. US—A—4,244,931, and US—A—4,247,526 disclose pyrophosphate salts in dicalcium phosphate systems. Jap. Patent Application Disclosure No. 4945—1974 discloses soluble pyrophosphates in a variety of dentifrice systems. US—A—4,333,551 discloses tetraalkali metal salts in mouthwash compositions. Finally Draus, Lesniewski and Miklos, *Pyrophosphate and Hexametalphosphate Effects In Vitro Calculus Formation*, Arch. Oral Biol., Vol. 15 pp 893—896, (1970) disclose the *in vitro* effectiveness of soluble pyrophosphate salts against calculus. However, they indicate that pyrophosphate would be inhibited by pyrophosphatase *in vivo*.

In spite of the many disclosures in the anticalculus and pyrophosphates areas, the need for an effective anticalculus product still exists. Surprisingly mixtures of certain pyrophosphate salts can provide a safe and effective product while also not presenting difficult formulation problems.

It is an object of the present invention to provide compositions which deliver an effective anticalculus benefit.

It is a further object of the present invention to provide an effective anticalculus product utilizing a mixture of soluble pyrophosphate salts.

These and other objects will become more clear from the detailed description which follows.

All percentages and ratios used herein are by weight unless otherwise specified.

The present invention relates to an oral composition in the form of a toothpaste comprising:

a) from 10% to 70% by weight of a dental abrasive selected from insoluble metaphosphates, alumina, thermosetting polymerized resins, and silica;

b) an amount of a fluoride ion source sufficient to supply from 50 ppm to 3500 ppm of fluoride ions;

c) an amount of a pyrophosphate salt selected from dialkali metal and mixtures of dialkali metal and tetraalkali metal pyrophosphate salts sufficient to provide at least 1.5% by weight of pyrophosphate ions $(P_2O_7^{-4})$; and

d) water

wherein the pH of the composition is from 6.0 to 10.0 and the composition does not contain more than 4.0% by weight of tetrapotassium pyrophosphate $(K_4P_2O_7)$.

The essential as well as optional components of the compositions of the present invention are described in the following paragraphs:

### Dental Abrasive

One class of abrasives for use herein are the particulate thermosetting polymerized resins as described by Cooley et al in US—A—3,070,510. Suitable resins include, for example, melamines, phenolics, ureas, melamine-ureas, melamineformaldehydes, urea-formaldehydes, melamine-urea-formaldehydes, cross-linked epoxides, and cross-linked polyesters.

Silica dental abrasives are also useful in the present compositions. The silica abrasive polishing material generally has an average particle size ranging between 0.1 and 30 µm, preferably 5 to 15 µm. The abrasive can be precipitated silica or silica gels such as the silica xerogels described in US—A—3,538,230. Preferred are the silica xerogels marketed under the tradename "Syloid®" by the W. R. Grace & Company, Davison Chemical Division. Also preferred are the precipitated silica materials such as those marketed by the J. M. Huber Corporation under the tradename, "Zeodent®". The types of silica dental abrasives useful in the toothpastes of the present invention are described in more detail in US—A—3,862,307.

Other suitable abrasives include alumina, and the insoluble metaphosphates such as insoluble sodium metaphosphate (IMP). Mixtures of abrasives can also be used. In any case, the total amount of abrasive in the dentifrice embodiments of this invention can range from 10% to 70% by weight of the dentifrice. Preferably, toothpastes contain from 10% to 50% by weight of abrasive.

The preferred abrasives are the silica abrasives since they are more compatible with the agents.

Fluoride Ion Source

The second essential component of the compositions herein is a fluoride ion source. The number of such sources is great and includes those disclosed in US—A—3,535,421. Typical materials include:

Stannous fluoride, potassium fluoride, lithium fluoride, cesium fluoride, ammonium fluoride, aluminum fluoride, cupric fluoride, indium fluoride, stannous fluorozirconate, lead fluoride, ferric fluoride, nickel fluoride, paladium fluoride, silver fluoride, zinc fluoride, zirconium fluoride, hexylamine hydrofluoride, laurylamine hydrofluoride, myristylamine hydrofluoride, decanolamine hydrofluoride, octadecenylamine hydrofluoride, myristoxyamine, hydrofluoride, diethylaminoethyloctoylamide hydrofluoride, diethanolamineoethyloleylamide hydrofluoride, diethanolaminopropyl-N'-octadecenylamine dihydrofluoride, 1-ethanol-2-hexadecylimidazoline dihydrofluoride, octoylethanolamine hydrofluoride, octyltrimethylammonium fluoride, dodecylethyldimethylammonium fluoride, tetraethylammonium fluoride, dilauryldimethylammonium fluoride, $\Delta^{8,9}$-octadecenylbenzyldimethylammonium fluoride, dioctyldiethylammonium fluoride, cyclohexylcetyldimethylammonium fluoride, furfuryllauryldimethylammonium fluoride, phenoxyethylcetyldimethylammonium fluoride, N:N'-tetramethyl-N:N;-dilaurylethylenediammonium difluoride, N-cetylpyridinium fluoride, N:N-dilauryl-morpholinium fluoride, N-myristyl-N-ethylmorpholinium fluoride, N-(octylaminocarbonylethyl)-N-benzyl-dimethylammonium fluoride, N(β-hydroxydodecyl)trimethylammonium fluoride, N-phenyl-N-hexadecyldiethylammonium fluoride, N-cyclohexyl-N-octadecyldimethylammonium fluoride, N-(2-carbomethoxyethyl)-N-benzyldimethylammonium fluoride, N-(2-carbocyclohexoxyethyl)-N-myristyldimethylammonium fluoride, N-(2-carbobenzyloxyethyl)-N-dodecyldimethylammonium fluoride, N-[2-(N:N'-dimethylaminocarbonyl)-ethyl]-N-dodecyldiethylammonium fluoride, N-carboxymethyl-N-cicosyldimethylammonium fluoride, betaine hydrofluoride, sarcosine stannous fluoride, alanine stannous fluoride, glycine potassium fluoride, sarcosine potassium fluoride, glycine hydrofluoride, lysine hydrofluoride, alanine hydrofluoride, betaine zirconium fluoride, sodium monofluorophosphate and mixtures thereof. Sodium fluoride is the preferred fluoride source.

The amount of the fluoride ion source should be sufficient to provide from 50 ppm to 3500 ppm, preferably from 500 ppm to 3000 ppm of fluoride ions.

Di alkali Metal and Tetra alkali Metal Salts

The pyrophosphate salts, useful in the present compositions are selected from dialkali metal pyrophosphate and mixtures of the dialkali metal and tetraalkali metal pyrophosphate salts. $Na_2H_2P_2O_7$, $Na_4P_2O_7$ and $K_4P_2O_7$ in their unhydrated as well as hydrated forms are the preferred species. The levels of each of these species which preferably are used in the compositions are as follows (all are in the unhydrated form).

| | |
|---|---|
| $Na_2H_2P_2O_7$ — | 0.5%—13.8% |
| $Na_4P_2O_7$ — | 0—6.0% |
| $K_4P_2O_7$ — | 0—4.0% |

The minimum amount of $P_2O_7^{-4}$ required in the present compositions, 1.5% is therefore provided solely by $Na_2H_2P_2O_7$ or mixtures of $Na_2H_2P_2O_7$ with either or both of the tetra alkali metal salts. Preferred are binary mixtures of the sodium salts and ternary mixtures of those with the tetra potassium salt. The upper limits on the sodium species are determined by solubility considerations while the tetra potassium level is established for taste reasons.

The pyrophosphate salts are described in more detail in Kirk & Othmer, *Encyclopedia of Chemical Technology*, Second Edition, Volume 15, Interscience Publishers (1968).

Water

Water is another essential component of the compositions of this invention. Water employed in the preparation of commercially suitable oral compositions should preferably be of low ion content and free of organic impurities. Water preferably comprises from 2% to 95%, more preferably from 2% to 45% of the compositions of this invention.

Optional Components

In addition to the above described essential components, the oral compositions of this invention can contain a variety of optional conventional oral composition components. Such optional ingredients include sudsing agents, flavoring agents, sweetening agents, antiplaque agents, coloring agents, and pigments.

A preferred optional ingredient is a sudsing agent. Suitable sudsing agents are those which are reasonably stable and form suds throughout a wide pH range, i.e., non-soap anionic, nonionic, cationic, zwitterionic and amphoteric organic synthetic detergents. Sudsing agents of these types are described more fully in US—A—3,959,458 and US—A—3,937,807.

Anionic sudsing agents useful herein include the water-soluble salts of alkyl sulfates having from 10 to 18 carbon atoms in the alkyl radical and the water-soluble salts of sulfonated monoglycerides of fatty acids having from 10 to 18 carbon atoms. Sodium lauryl sulfate and sodium coconut monoglyceride sulfonates are examples of anionic surfactants of this type. Mixtures of anionic surfactants can also be employed.

The nonionic sudsing agents which can be used in the compositions of the present invention can be broadly defined as compounds produced by the condensation of alkylene oxide groups (hydrophilic in nature) with an organic hydrophobic compound which may be aliphatic or alkylaromatic in nature. Examples of suitable nonionic sudsing agents include the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials.

The zwitterionic synthetic sudsing agents useful in the compositions of the present invention can be broadly described as derivatives of aliphatic quaternary ammonium, phosphonium, and sulfonium compounds, in which the aliphatic radicals can be straight chain or branched, and wherein one of the aliphatic substituents contains from 8 to 18 carbon atoms and one contains an anionic water-solubilizing group, e.g., carboxy, sulfonate, sulfate, phosphate, or phosphonate.

The cationic sudsing agents useful in the compositions of the present invention can be broadly defined as quaternary ammonium compounds having one long alkyl chain containing from 8 to 18 carbon atoms such as lauryl trimethylammonium chloride; cetyl pyridinium chloride, cetyl trimethylammonium bromide; di-isobutylphenoxyethoxyethyl-dimethylbenzylammonium chloride; coconutalkyltrimethyl-ammonium nitrite and cetyl pyridinium fluoride.

The amphoteric sudsing agents useful in the present invention can be broadly described as derivatives of aliphatic secondary and tertiary amines in which the aliphatic radical can be straight chain or branched and wherein one of the aliphatic substituents contains from 8 to 18 carbon atoms and one contains an anionic water-solubilizing group, e.g., carboxylate, sulfonate, sulfate, phosphate, or phosphonate.

The sudsing agent can be present in the compositions of this invention in an amount from 0% to 10% by weight of the total composition.

Flavoring agents can also be added to the instant compositions. Suitable flavoring agents include oil of wintergreen, oil of peppermint, oil of spearmint, oil of sassafras, and oil of clove. Sweetening agents which can be used include saccharin, dextrose, levulose, aspartame, D-tryptophan, dihydrochalcones, acesulfame and sodium cyclamate. Flavoring agents are generally used in the compositions at levels of from 0.4% to 2% by weight and sweetening agents at levels of from 0.1% to 5% by weight.

Binders can also be used with the toothpastes of the present inventions. Such binders include, for example, xanthan gum, carrageenan (Irish moss, Viscarin®), and carboxyvinyl polymers. These binders are generally present at a level of from 0.1% to 1%.

Bis-biguanide antiplaque agents can also optionally be added to the compositions of this invention. Such agents include chlorhexidine (1,6-bis[$N^5$-p-chlorophenyl-$N^1$-biguanido]hexane), the soluble and insoluble salts thereof and related materials such as 1,2-bis($N^5$-p-trifluoromethylphenyl-$N^1$-biguanido)-ethane are described more fully in US—A—3,923,002, US—A—3,937,807, BE—A—843,244, and BE—A—844,764.

If present, the optional antiplaque agents generally comprise from 0% to 5% by weight of the compositions herein.

Another optional component of the compositions herein is a humectant. The humectant serves to keep the toothpaste compositions from hardening upon exposure to air and in mouthwashes give a moist feel to the mouth. Certain humectants can also impart desirable sweetness of flavor to mouthwash and toothpaste compositions. The humectant, on a pure humectant basis, generally comprises from 0% to 70%, preferably from 0% to 55%, by weight of the compositions herein.

Suitable humectants for use in this invention include edible polyhydric alcohols such as glycerine, sorbitol, xylitol and propylene glycol. Sorbitol is frequently employed as a 70% aqueous solution known as Sorbo®.

The pH of the compositions herein is in the range of 6.0 to 10.0, preferably from 7.3 to 9.0. The pH is preferably achieved through a proper balancing of the pyrophosphate salts or by the addition of an alkaline or acidic agent.

Method of Manufacture

The compositions herein are made using conventional mixing techniques. A typical method is described in Example I.

Industrial Applicability

The compositions of the present invention are used in a conventional manner.

The following examples further describe and demonstrate preferred embodiments within the scope of the present invention. The examples are given solely for the purpose of illustration and are not to be construed as limitations of this invention.

Example I

The following is a toothpaste representative of the present invention.

| Component | % |
|---|---|
| Distilled Water | 16.484 |
| Sorbitol (70% Aqueous Solution) | 49.563 |
| Sodium Saccharin | 0.300 |
| Dye Solution | 0.350 |
| Precipitated Silica | 20.00 |
| Sodium Fluoride | 0.243 |
| Flavor | 1.330 |
| Sodium Alkyl Sulfate (27.9% Aqueous Solution) | 5.000 |
| Carbapol® 940s* | 0.180 |
| Xanthan Gum | 0.600 |
| $Na_4P_2O_7$ | 2.400 |
| $Na_2H_2P_2O_7$ | 1.190 |
| $K_4P_2O_7$ (63.5% Aqueous Solution) | 2.360 |
| | 100.000% |

*A carboxy vinyl polymer offered by B. F. Goodrich Company.

The above composition was made by combining the water and part of the sorbitol in an agitated mixture and heating this mixture to 60°C (140°F). The $Na_2H_2P_2O_7$, $Na_4P_2O_7$, saccharin, sodium fluoride and precipitated silica were then added in order and the total mixture was made for from 5 to 10 minutes. The flavor, dye and surfactant were then added. In a separate vessel the remainder of the sorbitol, the Carbopol® and the xanthan gum were slurried together and then added to the main mix tank. The complete batch was mixed for about one-half hour and subsequently milled and deaerated.

## Example II
The following is another representative toothpaste of the present invention.

| Component | % |
|---|---|
| Sorbitol (70% Aqueous Solution) | 50.743 |
| Distilled Water | 16.484 |
| Sodium Saccharin | 0.300 |
| Dye Solution | 0.350 |
| Precipitated Silica | 20.000 |
| Sodium Fluoride | 0.243 |
| Flavor | 1.330 |
| Sodium Alkyl Sulfate (27.9% Aqueous Solution) | 5.000 |
| Carbapol® 940s* | 0.180 |
| Xanthan Gum | 0.600 |
| $Na_4P_2O_7$ | 3.400 |
| $Na_2H_2P_2O_7$ | 1.370 |
| | 100.000% |

Both the composition of Example I and that of Example II are effective in reducing calculus and possess acceptable cosmetic properties.

In addition to the levels and combinations of ingredients shown in these examples, others can be used which are consistent with the invention disclosed and claimed herein.

**Claims**

1. An oral composition in the form of a toothpaste characterized in that it comprises:

a) from 10% to 70% by weight of a dental abrasive selected from insoluble metaphosphates, alumina, thermosetting polymerized resins, and silica;

b) an amount of a fluoride ion source sufficient to supply from 50 ppm to 3500 ppm of fluoride ions;

c) an amount of a pyrophosphate salt selected from dialkali metal and mixtures of dialkali metal and tetraalkali metal pyrophosphate salts sufficient to provide at least 1.5% by weight of pyrophosphate ions $(P_2O_7^{-4})$; and

d) water

wherein the pH of the composition is from 6.0 to 10.0 and the composition contains no more than 4.0% by weight of tetrapotassium pyrophosphate $(K_4P_2O_7)$.

2. An oral composition according to Claim 1 wherein the abrasive is a silica dental abrasive.

3. An oral composition according to Claim 2 comprising in addition from 0.1% to 1.0% by weight of a binder.

4. An oral composition according to any of Claims 1 to 3 wherein the fluoride ion source is sodium fluoride.

5. An oral composition according to any of Claims 1 to 4 wherein the pyrophosphate ion is provided by a mixture of disodium pyrophosphate and tetrasodium pyrophosphate.

6. An oral composition according to any of Claims 1 to 4 wherein the pyrophosphate ion is provided by a mixture of disodium pyrophosphate, tetrasodium pyrophosphate and tetrapotassium pyrophosphate.

7. An oral composition according to any of Claims 1 to 6 which in addition contains from 0 to 10% by weight of a sudsing agent and/or from 0 to 70% by weight of a humectant.

**Patentansprüche**

1. Eine Zusammensetzung für oralen Gebrauch in Form einer Zahnpaste, dadurch gekennzeichnet, daß sie enthält:

a) 10 Gew.-% bis 70 Gew.-% eines Zahnputzmittels, ausgewählt aus unlöslichen Metaphosphaten, Aluminiumoxid, wärmegehärteten, polymerisierten Harzen und Kieselsäure;

b) eine ausreichende Menge einer Fluoridionenquelle, um 50 TpM bis 3500 TpM Fluoridionen zu liefern;

c) eine zur Lieferung von wenigstens 1,5 Gew.-% an Pyrophosphationen ($P_2O_7^{-4}$) ausreichende Menge eines Pyrophosphatsalzes, das aus Dialkalimetall- und Gemischen von Dialkalimetall- und Tetraalkalimetallpyrophosphatsalzen ausgewählt ist; und

d) Wasser;

wobei der pH-Wert der Zusammensetzung 6,0 bis 10,0 beträgt, und die Zusammensetzung nicht mehr als 4,0 Gew.-% Tetrakaliumpyrophosphat ($K_4P_2O_7$) enthält.

2. Eine Zusammensetzung für oralen Gebrauch nach Anspruch 1, wobei das Putzmittel ein Dentalkieselsäureputzmittel ist.

3. Eine Zusammensetzung für oralen Gebrauch nach Anspruch 2, welche zusätzlich 0,1 Gew.-% bis 1,0 Gew.-% eines Bindemittels enthält.

4. Eine Zusammensetzung für oralen Gebrauch nach einem der Ansprüche 1 bis 3, wobei die Fluoridionenquelle Natriumfluorid ist.

5. Eine Zusammensetzung für oralen Gebrauch nach einem der Ansprüche 1 bis 4, wobei das Pyrophosphation durch ein Gemisch von Dinatriumpyrophosphat und Tetranatriumpyrophosphat geliefert wird.

6. Eine Zusammensetzung für oralen Gebrauch nach einem der Ansprüche 1 bis 4, wobei das Pyrophosphation durch ein Gemisch von Dinatriumpyrophosphat, Tetranatriumpyrophosphat und Tetrakaliumpyrophosphat geliefert wird.

7. Eine Zusammensetzung für oralen Gebrauch nach einem der Ansprüche 1 bis 6, welche zusätzlich 0 bis 10 Gew.-% eines schaumbildenden Mittels und/oder 0 bis 70 Gew.-% eines Feuchthaltemittels enthält.

**Revendications**

1. Composition orale se présentant sous la forme d'une pâte dentifrice, caractérisée par le fait qu'elle comprend:

a) de 10% à 70% en poids d'un abrasif dentaire choisi parmi les métaphosphates insolubles, l'alumine, les résines polymérisées thermodurcissables et la silice;

b) une quantité de source d'ions fluorure suffisante pour fournir de 50 ppm à 3500 ppm d'ions fluorure;

c) une quantité d'un sel de type pyrophosphate choisi parmi les sels de type pyrophosphates métalliques dialcalins et les mélanges de sels de type pyrophosphates métalliques dialcalins et métalliques tétraalcalins, suffisante pour fournir au moins 1,5% en poids d'ions pyrophosphate ($P_2O_7^{-4}$); et

d) de l'eau

dans laquelle le pH de la composition va de 6,0 à 10,0, et la composition ne contient pas plus de 4,0% en poids de pyrophosphate tétrapotassique ($K_4P_2O_7$).

2. Composition orale selon la revendication 1, dans laquelle l'abrasif est un abrasif dentaire de type silice.

3. Composition orale selon la revendication 2 comprenant, en outre, de 0,1 à 1,0% en poids d'un liant.

4. Composition orale selon l'une des revendications 1 à 3, dans laquelle la source d'ions fluorure est le fluorure de sodium.

5. Composition orale selon l'une des revendications 1 à 4, dans laquelle l'ion pyrophosphate est fourni par un mélange de pyrophosphate disodique et de pyrophosphate tétrasodique.

6. Composition orale selon l'une des revendications 1 à 4, dans laquelle l'ion pyrophosphate est fourni par un mélange de pyrophosphate disodique, de pyrophosphate tétrasodique et de pyrophosphate tétrapotassique.

7. Composition orale selon l'une des revendications 1 à 6, qui, en outre, contient de 0 à 10% en poids d'un agent moussant et/ou de 0 à 70% en poids d'un humectant.